# EUROPEAN PATENT APPLICATION

(11) **EP 3 964 209 A1**
(43) Date of publication of application: **09.03.2022**
(21) Application number: 19927857.3
(22) Date of filing: 07.05.2019
(51) Int. Cl.: A61K 31/353, A61K 31/5377, A61P 35/00

(54) **APPLICATION OF EPIGALLOCATECHIN GALLATE COMBINED WITH TYROSINE KINASE INHIBITOR IN PREPARATION OF CANCER TREATMENT DRUGS**

(71) Applicant: Yunnan Daye Dihong Biotechnology Co., Ltd., Kunming, Yunnan 650201 (CN); Yunnan Agricultural University, Kunming, Yunnan 650201 (CN)
(72) Inventor: SHENG, Jun, Kunming, Yunnan 650201 (CN); WANG, Xuanjun, Kunming, Yunnan 650201 (CN); HUANG, Yanping, Kunming, Yunnan 650201 (CN); ZI, Chengting, Kunming, Yunnan 650201 (CN); XIANG, Zemin, Kunming, Yunnan 650201 (CN); HUANG, Yewei, Kunming, Yunnan 650201 (CN); ZHAO, Yunli, Kunming, Yunnan 650201 (CN); CUAN, Xiangdan, Kunming, Yunnan 650201 (CN); XU, Huanhuan, Kunming, Yunnan 650201 (CN); LUO, Rui, Kunming, Yunnan 650201 (CN)
(74) Representative: Zaboliene, Reda
(86) International application number: PCT/CN2019/085776
(87) International publication number: WO 2020/223888

(57) **Abstract**

Disclosed is an application of Epigallocatechin Gallate (EGCG) or a compound synthesized with it as a lead compound in combination with one or more tyrosine kinase inhibitors in preparation of a cancer treatment drug, herein the cancer is an Epidermal Growth Factor Receptor (EGFR) wild-type tumor. This combined use may significantly inhibit the growth of the EGFR wild-type tumor, and reduce the toxic and side effects of an anticancer drug.

## Description

### Technical Field

The present invention belongs to the technical field of biomedicines, and specifically relates to an anticancer composition and an application thereof.

### Background

Tyrosine Kinase Receptor (TKR) Epidermal Growth Factor Receptor (EGFR) is widely expressed on various cell membranes except a suspension cell, the excessive activation of an EGFR protein is closely related to the occurrence, development, grade malignancy and prognosis of a tumor, and may cause tumor cell proliferation, and promote tumor tissue angiogenesis and tumor cell metastasis. EGFR is one of main target points for targeted therapy of a human tumor. A Tyrosine Kinase Inhibitor (TKI) is the main field of targeted therapy drug development. Gefitinib, developed for a wild-type EGFR tumor, is a first-generation TKI-type EGFR inhibitor, but it is found clinically that Gefitinib has no significant effect on a patient with highly activated EGFR after marketing. It is found from researches that the affinity of Gefitinib to EGFR with a L858R mutation is 5-6 times greater than that to a wild-type EGFR, and the effective rate thereof for a patient with this mutation is as high as 80%, it becomes a specific drug for these tumor patients. Patients with EGFRL858R account for only 6% of all tumor patients, and there are no effective TKI inhibitors that may be used clinically for more than 60% of EGFR wild-type patients. Therefore, it is of great significance to develop a new treatment strategy for the wild-type EGFR.

### Summary

In order to overcome problems in the background, there are no effective inhibitors for tumor patients with wild-type EGFR at present. The present invention provides a therapeutic method of a drug combination of EGCG or a synthetic compound designed by using the EGCG as part or all of a lead compound and one or more tyrosine kinase inhibitors. The combination of the two may improve the sensitivity of the wild-type EGFR to the original tyrosine kinase inhibitor, broaden a scope of application of the original inhibitor, and provide an effective treatment strategy for an EGFR wild-type cancer patient.

The present invention is achieved through the following technical schemes.

An application of EGCG combined with a tyrosine kinase inhibitor in preparation of a cancer treatment drug. The EGCG includes EGCG and a synthetic compound designed by using the EGCG as part or all of a lead compound, and the tyrosine kinase inhibitor includes a combination of one or more tyrosine kinase inhibitors.

Preferably, the cancer is a tumor expressing the wild-type EGFR.

Specifically, preferably the application of the EGCG combined with Gefitinib in preparation of an EGFR wild-type tumor therapeutic drug.

A pharmaceutical composition for treatment of a cancer, including a combination of an effective amount of the EGCG and one or more tyrosine kinase inhibitors, or including a combination of a synthetic compound designed by using the EGCG as part or all of a lead compound and one or more tyrosine kinase inhibitors.

Preferably, an application of the pharmaceutical composition is applied in preparation and treatment of an EGFR wild-type lung cancer drug.

Specifically, preferably the pharmaceutical composition for the treatment of the cancer includes EGCG and Gefitinib. While a drug for an EGFR wild-type tumor is prepared, the dosage of the compound may be adjusted according to a route of administration, age and weight of a patient, type and severity of a disease being treated and the like, the dosage of Gefitinib is 0.1-5 mg/kg in body weight, and the dosage of EGCG is 0.1-8 mg/kg in body weight.

The beneficial effects of the present invention are as follows.

The present invention discloses a combination of EGCG or a synthetic compound designed by using the EGCG as part or all of a lead compound and a tyrosine kinase inhibitor, especially the EGCG is combined with Gefitinib to prepare a pharmaceutical composition, and it may be used for the treatment of a patient with an EGFR wild-type tumor. It may be seen in vitro that the growth of a tumor cell is significantly inhibited, and the increase in tumor volume of EGFR wild-type tumor cell heterotransplantation in vivo may be inhibited. The combination of the two reduces the treatment risk and the toxic and side effects caused by the larger dosage of an anticancer drug.

The present invention not only provides an effective treatment strategy for the EGFR wild-type tumor patient, but also expands a scope of application of the original tyrosine kinase inhibitor.

### Brief Description of the Drawings

Fig. 1 is an effect of a combined use of EGCG and Gefitinib on proliferation of EGFR wild-type and mutant-type cells.
Fig. 2 is an effect of a combined use of EGCG and Gefitinib on a related protein in an EGFR signaling pathway in the EGFR wild-type and mutant-type cells.
Fig. 3 is an effect of a combined use of EGCG and Gefitinib on a body weight of an A431 tumor-bearing mouse.
Fig. 4 is a pictorial diagram of an effect of a combined use of EGCG and Gefitinib on tumor growth of an A431 transplanted tumor nude mouse.
Fig. 5 is an effect of a combined use of EGCG and Gefitinib on a tumor volume of the transplanted tumor nude mouse (transplanted tumor growth curve).
Fig. 6 is an effect of a combined use of EGCG and Gefitinib on a tumor weight of the A431 transplanted tumor nude mouse.
Fig. 7 is an effect of a combined use of EGCG and Gefitinib on a body weight of an NCI-H1975 tumor-bearing mouse.
Fig. 8 is a pictorial diagram of an effect of a combined use of EGCG and Gefitinib on tumor growth of an NCI-H1975 transplanted tumor nude mouse.
Fig. 9 is an effect of a combined use of EGCG and Gefitinib on a tumor volume of the NCI-H1975 transplanted tumor nude mouse (transplanted tumor growth curve).
Fig. 10 is an effect of a combined use of EGCG and Gefitinib on a tumor weight of the NCI-H1975 transplanted tumor nude mouse.

### Detailed Description of the Embodiments

In order to make the purposes, technical schemes and beneficial effects of the present invention clearer, preferred embodiments of the present invention are described in detail below, so that it is understood conveniently by those skilled in the art.

The present invention selects an EGFR wild-type and highly-expressed cell line and an EGFR double-mutant cell line as research objects. First-generation EGFR inhibitor Gefitinib mainly for EGCG and the EGCG are used in combination to inhibit the proliferation of an EGFR wild-type tumor cell and inhibit the tumor growth of a heterotransplanted nude mouse, and an action mechanism of this effect is elucidated.

### Embodiment 1: Effect of combined use of EGCG and Gefitinib on in vitro proliferation of EGFR wild-type and mutant-type cells

### 1. Experimental material

Cell line: Human epidermal squamous cell carcinoma A431, and human lung cancer cell lines NCI-H1666 and NCI-H1975.

### 2. Detection principle: MTT method to detect cell viability

Cell viability experiment detected by MTT: MTT concentration is 5 mg/ml. Therefore, 0.5 g of MTT may be weighed, dissolved in 100 ml of Phosphate Buffer Saline (PBS) or phenol red-free medium, and filtered with a 0.22 µm filter membrane to remove bacteria in solution. After the preparation is completed, it is sub-packaged and stored at 4°C in dark place. A container is best wrapped with aluminum foil paper.

Test procedure: A: An anchorage-dependent cell operation method is adopted, firstly logarithmic phase cells are collected to adjust the cell suspension concentration, so that the density of the cells to be tested is adjusted to 3×10⁴/well, and 200 µl is added per well (an edge well is filled with sterile PBS). At the same time, a zero adjustment well (dimethyl sulfoxide) and a control well (cells, the same concentration of a drug dissolving medium (acid medium), MTT, and dimethyl sulfoxide) are set, and cultured in an incubator of 5% CO₂ and 37°C.

B: After the cells are adhered to a wall, it may be seen that the cells cover the bottom of the well (96-well flat bottom plate) in monolayer, firstly the cells are washed with PBS, the PBS is discarded, and then dosing may start. 4-6 replicate wells are set according to 200 µl per well. Otherwise, it is difficult to reflect the real situation. After the treatment is completed, the same edge wells are filled with the acid medium. A sample is prevented from being volatilized.

C: It is incubated in 5% CO₂ and 37°C for 24 hours, and a cell state is observed under an inverted microscope.

D: 20 µl of MTT solution (5 mg/ml, namely 0.5% MTT) is added per well, and it continues to incubate for 4h. If the drug may react with MTT, the culture solution may be discarded after being centrifuged, after being carefully washed with PBS, the culture solution containing MTT is added again.

E: The culture is stopped, and the culture medium in the well is carefully absorbed.

F: 150 µl of the dimethyl sulfoxide is added per well, and it is placed on a shaker to shake at a low speed for 10 min, so that a crystal substance is fully dissolved. An absorbance value of each well is measured at OD490 nm of an enzyme-linked immunoassay. 630 nm is the absorbance value (OD value) detected at a reference point.

G: A cell viability calculation formula is as follows: viability=experimental group OD average value - blank group OD average value/1)×100%, obtained data obtains a column diagram in excel.

### 3. Experimental result

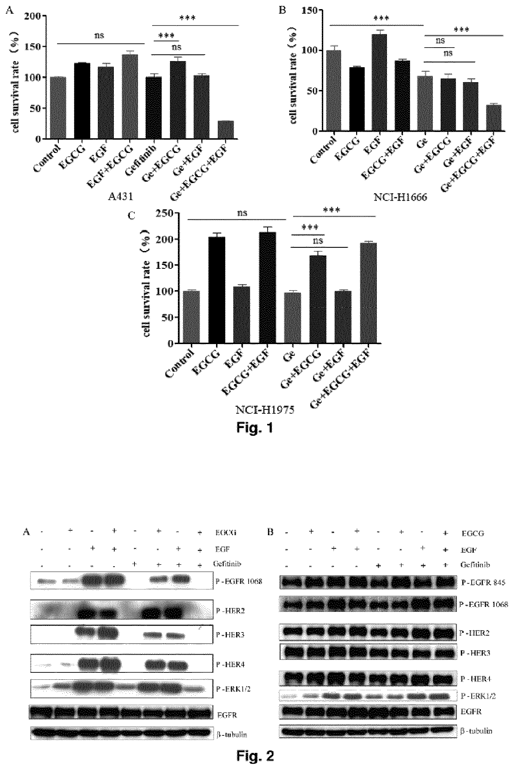

The results are shown in Fig. 1, a Gefitinib alone treatment group has no effect on the survival rate of A431 cells, and has a significant inhibitory effect on NCI-H1666 cells, but it is consistent with our expected results that Gefitinib combined with Epidermal Growth Factor (EGF) and EGCG treatment groups respectively does not have a significant inhibitory effect on the growth of the two wild-type EGFR cell lines. However, while Gefitinib is present in the presence of both EGCG and EGF, it may significantly inhibit the growth of the EGFR wild-type cells, but has no effect on double mutant NCI-H1975 cells. It preliminarily shows that this combined cell treatment mode has a selective inhibitory effect on the cell growth. It has a very significant inhibitory effect on the wild-type EGFR cells A431 and NCI-H1666, but has no effect on the double mutant NCI-H1975 cells.

### Embodiment 2: Effect of Gefitinib on phosphorylation of related protein in EGFR signaling pathway in wild-type and mutant-type cells in presence of both EGCG and EGF

1. Experimental material
   Cell line: Human epidermal squamous cell carcinoma A431, and a human lung cancer cell line NCI-H1975.
2. Detection principle: Change conditions of related protein in cells detected by Western Blot
3. Experimental method: a: The cells are treated, starvation treatment is performed after the cells are adhered to a wall, and after overnight treatment, they are treated with EGCG and EGF. Experiments are performed according to specific experimental grouping, a blank control group is directly added with an acid medium, and the rest of experimental groups are added with 10 mL of the acid medium, so that the final concentration of EGCG is 20 µg/mL, and EGF mother liquor is diluted to 20 ng/mL and added to the acid medium. After dosing is completed, it is placed in a cell incubator, and corresponding treatment time is set according to the experimental group.
   b: A protein is extracted.
   c: Protein quantification: A principle of protein determination uses a BCA protein quantification method.
   d: Based on a principle of SDS-PAGE, proteins of different molecular weights are separated by the electric field strength according to the different electric charge quantities carried by the different proteins. Then the separated protein is transferred to a PVDF membrane, and the protein is adsorbed in the form of a non-covalent bond without destroying the biological activity of a protein polypeptide. Then the protein transferred to the membrane is used as an antigen, and a corresponding antibody is combined with the antigen overnight or two hours at a room temperature, and then combined with an HRP-labeled secondary antibody at the room temperature for 1 hour. A target protein may be detected after substrate color developing.
4. Experimental result
   The results are shown in Fig. 2: In wild-type EGFRA431 cells, in the presence of both EGCG and EGF, Gefitinib may significantly inhibit the phosphorylation of an EGFR family and a downstream protein ERK thereof. However, this result is not seen in double mutant EGFR NCI-H1975 cells. The experiment results are consistent with the cell survival rate experiment.

### Embodiment 3: Inhibitory effect of combined use of EGCG and Gefitinib on tumor in EGFR wild-type AA431 cell carcinoma heterotransplanted tumor model

### 1. Experimental material

A431 cells and culture: Human epidermal squamous cell carcinoma A431 is cultured in a DMEM high-sugar complete medium containing 10% FBS, and the cells are diluted to 2 × 106 cells per mL of culture solution. It is inoculated into a culture dish. It is placed in a cell incubator for culture. The cells in a logarithmic growth phase are taken, and digested with 0.5% pancreatin solution to prepare a single cell suspension, it is centrifuged at 15000 rpm for 3 min, a supernatant is discarded, and the cell density is adjusted to 5×10⁶/mL with PBS.

Nude mouse and rearing thereof: A male BALB/C nu/nu clean-grade mouse, 6-8 weeks old, is provided by Changzhou Cavens Laboratory Animal Co., Ltd., Jiangsu Province. The nude mouse is kept in a sterile and ventilated closed animal room at a temperature of 25°C, with regular light during the day and a dark environment at night, and with free food and drinking water. Cage and water of the nude mouse are sterilized by high temperature and high pressure. Clean cage, padding, drinking water and the like are regularly changed for the nude mouse, to keep the growth environment of the nude mouse clean.

### 2. Experimental method

a: Establishment of nude mouse transplanted tumor model: Cells are resuscitated and passaged. After one week, a cell state is adjusted, and the cell growth environment tends to be stable. After the cells are digested and counted, the density of viable cells is adjusted to 5×10⁶ cells per 200µL of suspension for later use. Then it is centrifuged at 1500g for 3 min. After the medium is absorbed, it is gently blown evenly with physiological saline to prepare a cell suspension for later use. In a sterile super clean bench, nude mice are grouped according to a principle that average values of body weights are consistent and labeled by cutting ears, and 200µL of the cell suspension (about 5×10⁶ cells) is subcutaneously injected into the back of each nude mouse. Every two days, a vernier caliper is used to measure the long diameter (Dmax) and the short diameter (Dmin) of the tumor, and the tumor volume V (V = Dmax × Dmin2/2) is calculated.

b: Animal grouping and administration: The nude mice are administered according to the following grouping: (1) Blank control group: physiological saline is intraperitoneally injected every six days in a week, and physiological saline containing 1%-Tween 80 is intragastrically administered; (2) Gefitinib group: it is intragastrically administered at the dosage of 50 mg/kg (twice a week); (3) EGCG group: it is administered once a day in six days of a week (intraperitoneal injection), and the dosage is 20 mg/kg or 40 mg/kg respectively; and (4) combined administration group: it is administered through an administration mode of the intraperitoneal injection every six days in a week (once a day), the dosage is 20 mg/kg or 40 mg/kg respectively, and the experiment is performed by administering Gefitinib at the administration dosage of 50 mg/kg twice a week. The tumor volume is measured before administration, three times a week. In addition, the body weight of the nude mouse is weighed, it is performed once a week, and an experiment record is made. After the administration is completed, the nude mouse is killed, and a tumor mass is taken out and photographed. A solid tumor is saved according to subsequent experimental arrangements.

### 3. Experimental result

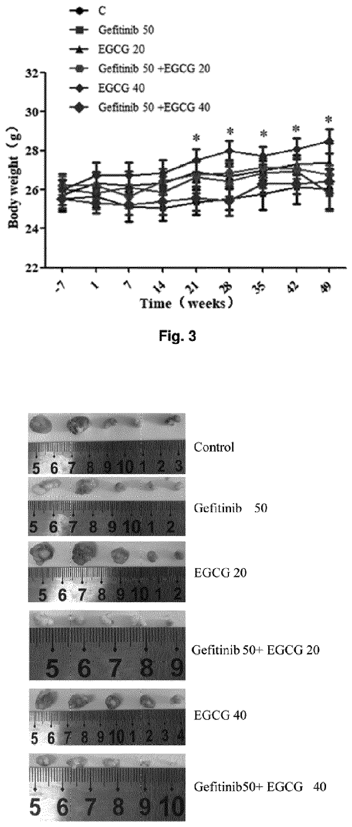

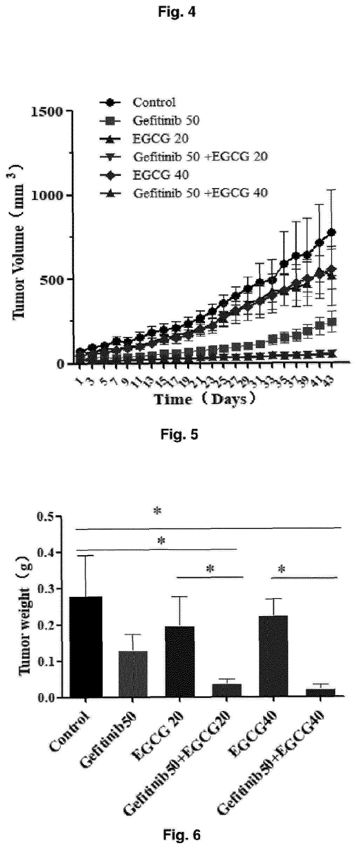

As shown in Fig. 3, it is found that compared with an animal in the normal control group, there is no significant weight loss between the groups. However, the EGCG alone treatment group may promote the weight increase of the nude mouse after 3 weeks of the administration, and it is significant compared with the control group. It shows that the drug in each group has no apparent toxic and side effects on the animals. After the administration is completed, the tumor of the nude mouse is taken out. As shown in Fig. 4, it shows the effect of the combined use on the tumor growth. A tumor growth curve is drawn by measuring the transplanted tumor in the nude mouse every two days. As shown in Fig. 5, compared with the Gefitinib alone treatment group, the Gefitinib combined use group significantly inhibits the tumor volume growth from the 29-th measurement of the tumor volume; and consistent with previous experimental results, Gefitinib has the better selectivity in vitro than the experiment in vivo. This experiment also shows that Gefitinib has the better inhibitory effect on the growth of the tumor in the nude mouse. While combined with EGCG, it may show a very apparent inhibitory effect, and the growth of tumor cells is basically completely inhibited. The tumor is taken out and weighed. The results are shown in Fig. 6. Compared with the blank control group, the tumor inhibition rate of the Gefitinib alone treatment group is 52%. The Gefitinib combined use group is compared with the Gefitinib alone treatment group, the inhibition rates of the solid tumor are 69% and 81% respectively. There is no statistical difference, but the combined use group inhibits the tumor growth to a certain extent compared to the single use. No animal dies during the whole experiment process.

### Embodiment 4: Inhibitory effect of combined use of EGCG and Gefitinib on tumor in EGFR double mutant NCI-H1975 cell carcinoma heterotransplanted tumor model

### 1. Experimental material

NCI-H1975 cells and culture: Human non-small cell lung cancer NCI-H1975 is cultured in a DMEM1640 complete medium containing 10% FBS, and the cells are diluted to 2 × 10⁶/mL. It is inoculated into a culture dish. It is placed in a cell incubator for culture. The cells in a logarithmic growth phase are taken, and digested with 0.5% pancreatin solution to prepare a single cell suspension, it is centrifuged at 15000 rpm for 3 min, a supernatant is discarded, and the cell density is adjusted to 5 × 10⁶/mL with PBS.

Nude mouse and rearing thereof: A male BALB/C nu/nu clean-grade mouse, 6-8 weeks old, is provided by Changzhou Cavens Laboratory Animal Co., Ltd., Jiangsu Province. The nude mouse is kept in a sterile and ventilated closed animal room at a temperature of 25°C, with regular light during the day and a dark environment at night, and with free food and drinking water. Cage and water of the nude mouse are sterilized by high temperature and high pressure. Clean cage, padding, drinking water and the like are regularly changed for the nude mouse, to keep the growth environment of the nude mouse clean.

### 2. Experimental method

a: Establishment of nude mouse transplanted tumor model: Cells are resuscitated and passaged. After one week, a cell state is adjusted, and the cell growth environment tends to be stable. After the cells are digested and counted, the density of viable cells is adjusted to 3 × 10⁶ cells per 200µL of suspension for later use. Then it is centrifuged at 1500g for 3 min. After the medium is absorbed, it is gently blown evenly with physiological saline to prepare a cell suspension for later use. In a sterile super clean bench, nude mice are grouped according to a principle that average values of body weights are consistent and labeled by cutting ears, and 200µL of the cell suspension (about 3 × 10⁶ cells) is subcutaneously injected into the back of each nude mouse. Every two days, a vernier caliper is used to measure the long diameter (Dmax) and the short diameter (Dmin) of the tumor, and the tumor volume V (V = Dmax × Dmin2/2) is calculated.

b: Animal grouping and administration: From the second day after the tumor cells are inoculated, the nude mice are administered according to the grouping: (1) Blank control group: physiological saline is intraperitoneally injected every six days in a week, and physiological saline containing 1%-Tween 80 is intragastrically administered; and Gefitinib is dissolved in 1%-Tween 80 to prepare a suspension; (2) Gefitinib group: Gefitinib is intragastrically administered twice a week at the dosage of 50 mg/kg or 100 mg/kg; (3) EGCG group: EGCG is administered once a day in six days of a week, and the dosage of intraperitoneal injection is 40 mg/kg; and (4) combined administration group: EGCG is administered once a day in six days of a week at the dosage of 40 mg/kg and Gefitinib is administered twice a week at the dosage of 50 mg/kg or 100 mg/kg. The tumor volume measurement is performed every two days, and the body weight of the nude mouse is weighed every three days, and recorded. After the administration is completed, the nude mouse is killed, and the tumor is taken out and photographed. A solid tumor is saved according to subsequent experimental arrangements. In a modeling experiment, 6-8-week-old nude mice are selected, and divided into 6 groups, each group has 5-6 mice, and they are fed with sterile feed.

### 3. Experimental result

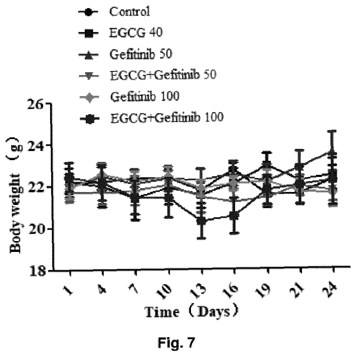

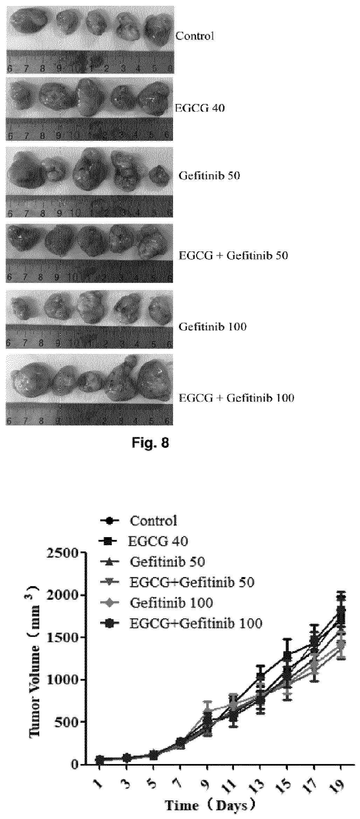

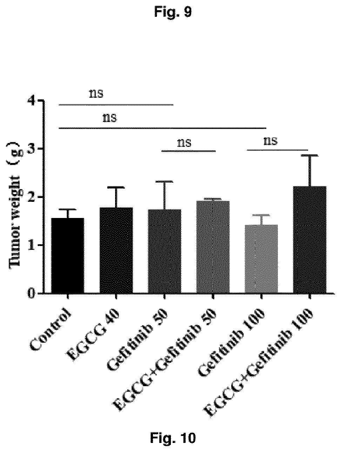

The administration is started on the second day after the tumor cells are injected, the body weight of the nude mouse is measured every 3 days during the administration period. The results are shown in Fig. 7: There is no difference in the effect of each group of the drugs on the body weight of the NCI-H1975 tumor-bearing mouse, and there is no death of the nude mouse caused by the drug during the whole process, it is indicated that the drug has no toxic and side effects on the nude mouse. The inhibitory effect of each group of the drugs on the tumor growth of the NCI-H1975 tumor-bearing mouse is as shown in Fig. 8, it may be seen from the figure that after the combined administration of Gefitinib and EGCG, the tumor growth of the NCI-H1975 tumor-bearing nude mouse is not inhibited, and there is no difference in tumor growth between the groups. The results show that this combined use has no effect on the EGFR double-mutant cell NCI-H1975 transplanted tumor, and it is further proved that this combined use only has the inhibitory effect on the EGFR wild-type tumor. The tumor volume of the transplanted tumor nude mouse is measured every two days, and a tumor growth curve is drawn. The results are shown in Fig. 9, and the combined use has no effect on the tumor volume of NCI-H1975 cell transplanted tumor; and the peeled tumor is weighed, the results are shown in Fig. 10, and the combined use has no effect on the tumor weight of the EGFR double mutant 1975 cell transplanted tumor.

The present invention selects the EGFR wild-type and highly-expressed epidermal squamous cell line and the EGFR double mutant lung cancer cell line as controls. It is provided from a series of cell experiments (MTT, Western-blotting) that after Gefitinib and EGCG and derivatives thereof are used in combination, it shows very apparent inhibition on the EGFR wild-type tumor cells, but has no effect on EGFR double mutant non-small cell lung cancer. This shows that this drug combination is selective.

It is further proved from in vivo experiments that the combined use of Gefitinib and EGCG may significantly inhibit the growth of EGFR wild-type tumor cell transplanted tumor, and has no significant effect on the body weight, it is indicated that this combination has no apparent toxic and side effects.

In summary, the combined use of EGCG and EGFR inhibitor Gefitinib may significantly inhibit the growth of the EGFR wild-type tumor cells without causing harm to the body. The combination of the two reduces the treatment risk and the toxic and side effects caused by the larger dosage of an anticancer drug. The present invention provides an effective treatment strategy for the EGFR wild-type tumor patient, improves the sensitivity of the original inhibitor, and broadens a scope of application of the original inhibitor.

While the drug for the EGFR wild-type lung cancer is prepared, the dosage of the compound may be adjusted according to a route of administration, age and weight of a patient, type and severity of a disease being treated and the like, the dosage of Gefitinib is 0.1-5 mg/kg in body weight, and the dosage of EGCG is 0.1-8 mg/kg in body weight.

Finally, it is to be noted that the above preferred embodiments are only used to illustrate the technical schemes of the present invention and not to limit. Although the present invention is already described in detail through the above preferred embodiments, those skilled in the art should understand that various changes may be made to it in forms and details without departing from a scope defined by the claims of the present invention.

## Claims

1. An application of epigallocatechin gallate (EGCG) combined with a tyrosine kinase inhibitor in preparation of a cancer treatment drug, wherein: the EGCG comprises EGCG and a synthetic compound designed by using the EGCG as part or all of a lead compound, and the tyrosine kinase inhibitor comprises a combination of one or more tyrosine kinase inhibitors.

2. A pharmaceutical composition for treatment of a cancer, wherein it comprises a combination of an effective amount of the EGCG and one or more tyrosine kinase inhibitors as claimed in claim 1, or comprises a combination of a synthetic compound designed by using the EGCG as part or all of a lead compound and one or more tyrosine kinase inhibitors.

3. The application as claimed in claim 1, wherein the cancer is an Epidermal Growth Factor Receptor (EGFR) wild-type tumor.

4. An application of the pharmaceutical composition as claimed in claim 2 in preparation and treatment of an EGFR wild-type tumor drug.
